# EUROPEAN PATENT APPLICATION

(11) **EP 3 751 333 A1**
(43) Date of publication of application: **16.12.2020**
(21) Application number: 19751186.8
(22) Date of filing: 06.02.2019
(51) Int. Cl.: G02C 13/00, G02C 7/02, G02C 9/00, G02F 1/13, G02F 1/1333

(54) **EYEWEAR SYSTEM**

(30) Priority: 07.02.2018 JP 2018020329; 19.10.2018 JP 2018197410
(71) Applicant: Mitsui Chemicals, Inc., Minato-ku Tokyo 105-7122 (JP)
(72) Inventor: HIKOSAKA, Eiichiro, Aichi 457-8522 (JP); KAN, Ryuki, Aichi 457-8522 (JP); MURAMATSU, Akihiro, Aichi 457-8522 (JP); OKADA, Yoshinobu, Aichi 457-8522 (JP); ASADA, Hirofumi, Aichi 457-8522 (JP)
(74) Representative: Epping - Hermann - Fischer
(86) International application number: PCT/JP2019/004293
(87) International publication number: WO 2019/156130

(57) **Abstract**

This eyewear system is provided with a first lens including an optical characteristic variation region in which an optical characteristic varies, a first frame for retaining the first lens, and a second frame for retaining a second lens different from the first lens, the first frame having a first attachment part for attaching the first frame to the second frame, or the second frame having a second attachment part for attaching the second frame to the first frame, so that the first lens and the second lens face each other and the optical characteristic variation region is positioned in the field of view of a subject through the second lens.

## Description

### Technical Field

The present invention relates to an eyewear system using eyewear having a lens including an optical characteristics change region.

### Background Art

Recently, eyewear with a lens having an optical characteristic change region in which the optical characteristics are changed by electrical control and/or the like has been developed. Specifically, for example, when a user operates a switch provided in the eyewear, electricity is supplied to the optical characteristic change region, the optical characteristics of the region, for example, the refractive index, the color, the polarization state, and/or the like are changed. As an example of such an eyewear, there is an electronic glasses disclosed in PTL1.

### Citation List

### Patent Literature

PTL1
Japanese Patent Application Laid-Open No.2009-98649

### Summary of Invention

### Technical Problem

Aviewing way of electronic glasses varies greatly depending on a change in optical characteristics. For this reason, there is a demand to allow a new user who has not used electronic glasses to experience with a difference in the viewing way due to the change in optical characteristics. However, the visual acuity of the user who intends to experience electronic glasses is not uniform. It is difficult to prepare in advance electronic glasses that can cope with the visual acuity of all the users. For this reason, there is a demand for a mechanism capable of easily experiencing the difference in the viewing way due to the change in optical characteristics for various users having different visual acuities.

As an example of such a mechanism, there is a configuration in which a first lens having an optical characteristic change region and a second lens having a diopter suited to the visual acuity of the user are separately prepared, and the first lens and the second lens are arranged in front of the eye of the user in a state of being overlapped with each other.

In order to give the user suitable experience with the eyewear provided with the lens having the optical characteristic change region, it is necessary to position the optical characteristic change region at a suitable position in the field of view of the user. Specifically, the preferred position is, for example, a lower position in the field of view of the user, when the lens having an electrical element is a bifocal lens (the region of the electrical element is for short distance, the other region is for long distance).

However, when the first lens and the second lens are arranged in front of the user's eye in the overlapped state, the optical characteristic change region may not be the suitable position.

In view of the above circumstances, it is an object of the present invention to provide the eyewear system capable of adjusting the relative position between the lens with the optical characteristic change region and the lens without the same.

### Solution to Problem

Eyewear system according to the present invention includes: a first lens including an optical characteristic change region with varying optical characteristics; a first frame holding the first lens; and a second frame holding a second lens different from the first lens, wherein the first frame has a first attaching portion for attaching the first frame to the second frame, or the second frame has a second attaching portion for attaching the second frame to the first frame, such that the first lens and the second lens face each other, and the optical characteristic change region is located within a field of view of an experiencing person through the second lens.

### Advantageous Effects of Invention

According to the present invention, it is possible to adjust the relative position between the lens with the optical characteristic change region and the lenses without the same.

### Brief Description of Drawings

FIG. 1 is a perspective view illustrating an example of a configuration of an eyewear system according to Embodiment 1 of the present invention;
FIG. 2 is a perspective view illustrating an example of the configuration of electronic glasses (the first frame of the present invention);
FIG. 3 is a block diagram illustrating a functional configuration of electronic glasses;
FIG. 4A is a diagram for explaining lens holder;
FIG. 4B is a diagrams for explaining lens holder;
FIG. 5 is a diagram for explaining lens unit;
FIG. 6A is a perspective view illustrating lens holder in the case that two sets of first supporting portion and second supporting portion are provided along the front-back direction;
FIG. 6B is a diagram illustrating a condition in which two lens units are inserted into lens holder in which two sets of first supporting portion and second supporting portion are provided along the front-back direction;
FIG. 7 is a diagram illustrating a state in which lens unit 300 suited to the visual acuity of the experiencer is attached to lens holder;
FIG. 8A is a diagram illustrating a state of positional adjustment of lens unit as viewed from the front side;
FIG. 8B is a diagram illustrating a relation between the positional adjustment of lens unit and the field of view of the experiencer as viewed from the lateral side;
FIG. 9A is a diagram illustrating an example in which the lens holder adjusts the lens of the lens unit in the yaw direction according to the front shape (curve) of the electronic glasses;
FIG. 9B is a diagram illustrating an example in which the lens holder adjusts the lens the lens unit in the pitch direction according to the front shape (inclination) of the electronic glasses;
FIG. 10 is a perspective view from the front of overglass (the first frame of the present invention);
FIG. 11 is a perspective view from the rear of overglass;
FIG. 12A is a perspective view illustrating a state before overglass is attached to experient's glasses;
FIG. 12B is a perspective view illustrating a state after overglass is attached to experient's glasses;
FIG. 13A is a diagram illustrating a state before the position adjustment in the front-back direction is performed after overglass is attached to experient's glasses;
FIG. 13B is a diagram illustrating a state in which the position adjustment is performed so that electronic lens approaches the lens of experient's glasses in the front-back direction of overglass in a state in which overglass is attached to experient's glasses;
FIG. 14A is a diagram illustrating a state before the position adjustment in the left-right direction is performed after overglass is attached to experient's glasses
FIG. 14B is a diagram illustrating a state in which the position of overglass is adjusted in the left-right direction in a state in which overglass is attached to experient's glasses;
FIG. 15A is a diagram illustrating a state before the position adjustment in the up-down direction is performed after overglass is attached to experient's glasses; and
FIG. 15B is a diagram illustrating a state in which the position adjustment is performed in the up-down direction of overglass in a state in which overglass is attached to experient's glasses.

### Description of Embodiments

### (Embodiment 1)

Hereinafter, an eyewear system according to Embodiment 1 of the present invention will be described with reference to the drawings.

### [Configuration of Eyewear System]

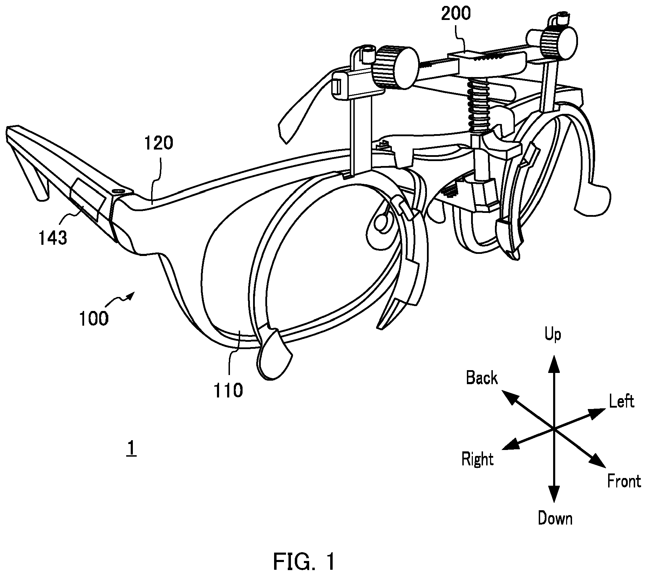

FIG. 1 is a perspective view illustrating an example of a configuration of an eyewear system 1 according to Embodiment 1 of the present invention. As illustrated in FIG. 1, eyewear system 1 has electronic glasses 100 and lens holder 200. Electronic glasses 100 are an example of the first frame of the present invention. Lens holder 200 is an example of the second frame of the present invention.

Electronic glasses 100 are an eyewear having electronic lens 110 including an optical characteristics change region change region in which optical characteristics are changed by electronic control and a frame 120 holding electronic lens 110. In the present invention, the eyewear is a device worn to hold an auxiliary mechanism for improving vision or field of view and a mechanism for presenting information to the eye, the eyewear is not limited to the eyeglasses-type worn on both ears, the eyewear may be a device worn on the head or one ear only. The eyewear may not affect both eyes but only one eye. The eyewear of the present invention includes various devices having a mechanism for presenting information to a user's field of view or eyes (e.g., a glasses-type wearable terminal, a head-mounted display, and/or the like.).

In Embodiment 1 described below, electronic glasses 100 for both eyes having a pair of lenses will be described as an example of the first frame of the present invention, however, the first frame of the present invention is not limited thereto.

Note that the front-back direction, the left-right direction, or the up-down direction correspond to the front-back direction, the left-right direction, or the up-down direction for the user wearing electronic glasses 100. In Embodiment 1, eyewear system 1 will be described using the front-back direction, the left-right direction, and the up-down direction illustrated in FIG. 1.

### [Configuration of Electronic Glasses 100]

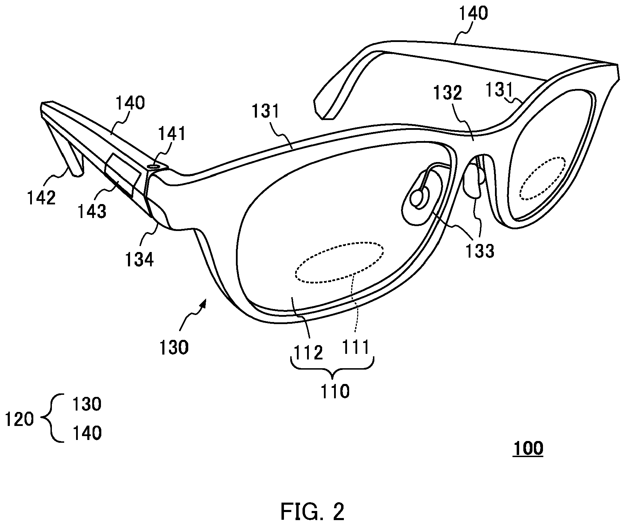

FIG. 2 is a perspective view illustrating an example of the configuration of electronic glasses 100. As described above, electronic glasses 100 have a pair of electronic lenses 110 and frame 120. Frame 120 has front 130 and a pair of temples 140. Electronic lens 110 is an example of the first lens of the present invention.

A pair of electronic lenses 110 are formed so as to be bilaterally symmetrical when viewed from the front of electronic glasses 100, and have the same components to each other.

Electronic lens 110 has a first region 111 in which the optical characteristics can be changed by electronic control and/or the like, and a second region 112 which is a region other than first region 111. First region 111 is an example of the optical characteristic change region of the present invention. Examples of optical characteristics that vary in first region 111 include refractive index, color, polarization state, light transmittance, and/or the like. In Embodiment 1, second region 112 of electronic lens 110 other than first region 111 is a region in which the optical characteristics do not change.

The shape, the size, and the position relative to the entire electronic lens 110 of first region 111 may be appropriately designed depending on the size of electronic lens 110, the application of electronic glasses 100, and/or the like. Examples of the shape of first region 111 include a circular shape, an elliptical shape, and/or the like. In Embodiment 1, the shape of first region 111 is an elliptical shape whose major axis is the left-right direction of electronic glasses 100. As illustrated in FIG. 2, when viewing electronic lens 110 in front, first region 111 is disposed below the central portion of electronic lens 110.

The structure of electronic lens 110 is as follows, for example. The structure in first region 111 of electronic lens 110 is a multilayer structure, having at least a liquid crystal layer (not illustrated), a pair of conductive layers sandwiching the liquid crystal layer from front and rear (not illustrated). Meanwhile, the structure in second region 112 of electronic lens 110 is configured by, for example, a spherical lens of a predetermined diopter (including the case without optical correction), or configured by an aspherical lens. As described above, electronic lens 110 is configured to have first region 111 and second region 112 by combining different structures. The diopter of first region 111 in the state where the optical characteristics are not changed may be the same as the diopter of second region 112, or may be a predetermined diopter different from the diopter of second region 112.

In second region 112, a pair of electrodes for supplying electricity to first region 111 (not illustrated) is provided inside of electronic lens 110, each electrode is connected to first region 111. A pair of electrodes is connected to battery 160 described below through wires disposed in frame 120 and/or the like. When a voltage is applied between a pair of conductive layers, the liquid crystal layer is activated and the optical characteristics of first region 111 are changed. Examples of electrodes include transparent electrodes such as ITO.

In Embodiment 1, the case where the optical characteristics of second region 112 other than first region 111 of electronic lens 110 do not change is described. However, the present invention may include the case where the optical characteristics of second region 112 change. In this case, it is desirable that the change in the optical characteristic in second region 112 is a change different from the change in the optical characteristic in first region 111.

As illustrated in FIG. 2, front 130 has a pair of rims 131 each supporting a pair of electronic lenses 110, and a bridge 132 connecting a pair of rims 131 to each other. Front 130 is an example of the first front frame of the present invention. The shape of rim 131 is a shape corresponding to the shape of electronic lens 110. Bridge 132 has a pair of nasal pads 133 that can contact the user's nose. Although not illustrated in particular, wiring for electrically connecting the electrode of electronic lens 110 and control unit 150 described later are disposed inside of front 130.

Front 130 has an end piece (closing block) 134 near both ends thereof. End piece 134 extends leftward and rearward, or rightward and rearward as viewed from rim 131. The rear tip of end piece 134 is connected to temple 140 by a hinge 141. Temple 140 is an example of the first temple of the present invention.

The material of front 130 is not particularly limited. It is preferable that the material of front 130 is a material having thermoplasticity and being capable of adjusting the position and the shape of each portion if necessary. As the material of front 130, a known material which is used as a material of the front of the glasses may be used. Examples of the material of front 130 include polyamide, acetate, carbon, celluloid, polyetherimide, and urethane.

A pair of right and left temples 140 are formed so as to have a nearly bilaterally symmetrical external shape in electronic glasses 100. As illustrated in FIG. 2, temple 140 is rotatably connected to front 130 at a hinge 141 of a front end of temple 140. The term "front" or "rear" in the following description of temple 140 means front or rear of temple 140 in the unfolded state (illustrated in FIG. 2 and/or the like).

The material of temple 140 is not particularly limited. However, it is preferable that the material of temple 140 is a material having thermoplasticity and being capable of adjusting the position and shape of each portion if necessary. As an example of the material of temple 140, a known material which is used as a material of a temple of general glasses, for example, the same material as an example of a material of front 130, may be used.

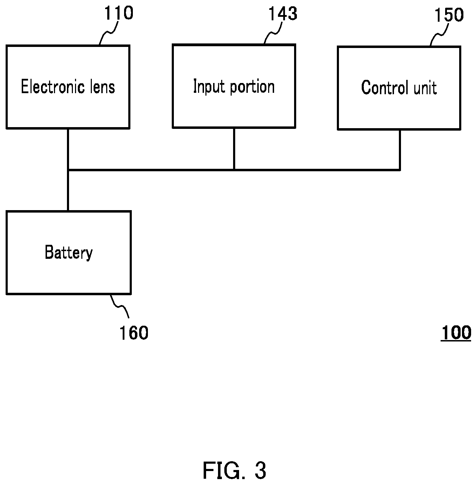

An ear hooking portion 142 is formed in the vicinity of the rear end portion of temple 140. Battery 160 (see FIG. 3 below) for supplying electricity to electronic lens 110 is attached at the rear end of ear hooking portion 142, although not illustrated in FIG. 2. Note that other electronic components may be attached to the rear end portion of ear hooking portion 142 in addition to battery 160. Examples of the other electronic components include memories for storing various types of data, transceivers for radio communications (Wi-Fi (registered trademark), Bluetooth (registered trademark), NFC, and/or the like), cameras, microphones, bone conducting speakers, hearing aids, and/or the like.

As illustrated in FIG. 2, input portion 143 is provided near the front end of temple 140. Input portion 143 is, for example, a portion that can be touched by a finger and/or the like of the user for operation of electronic glasses 100. As illustrated in FIG. 2, at least a part of input portion 143 is disposed so as to be exposed to the outside of temple 140.

Input portion 143 is preferably disposed at a position where the user of electronic glasses 100 can easily touch input portion 143. From this point of view, input portion 143 is disposed on the front side of the midpoint in the long axis direction of temple 140. Input portion 143 is disposed on the outer surface of temple 140 when viewed from the user of electronic glasses 100. Although input portion 143 in FIG. 2 is provided on the right side of temple 140, input portion 143 may be provided on the left side of temple 140, or may be provided on both.

The shape of input portion 143 is not particularly limited. In Embodiment 1, the input portion 143 is formed in a laterally long and nearly rectangular shape, and extends along the long axis direction of temple 140.

Input portion 143 is, for example, a capacitive touch sensor, and is connected to control unit 150 as illustrated in FIG. 3 below. Input portion 143 has conductivity, when an object (such as the user's finger) which is a conductor is in touch with input portion 143, control unit 150 can detect the touch. Examples of materials for input 143 include gold, silver, copper, aluminum and alloys thereof. In this case, it is preferable that the material of at least the peripheral portion of the input portion 143 (for example, temple 140) is insulating. Input portion 143 is not limited to the capacitive touch sensor, and may be a mechanical switch, for example.

Control unit 150 is configured by, for example, a CPU and/or the like, and controls whether or not the optical characteristic of first region 111 of electronic lens 110 is changed. FIG. 3 is a block diagram illustrating a functional configuration of electronic glasses 100. As illustrated in FIG. 3, in electronic glasses 100, electronic lens 110, input portion 143 of temple 140, control unit 150, and battery 160 are electrically connected.

Control unit 150 detects a change in capacitance in input portion 143, when detected, applies a voltage to first region 111 of electronic lens 110. Specifically, for example, when input portion 143 detects the contact of the object, control unit 150 applies a voltage to first region 111 or executes control to stop the application of the voltage, and changes the optical characteristics of first region 111.

Although not illustrated, control unit 150 may be accommodated, for example, in the vicinity of the front end portion of temple 140.

### [Lens Holder 200]

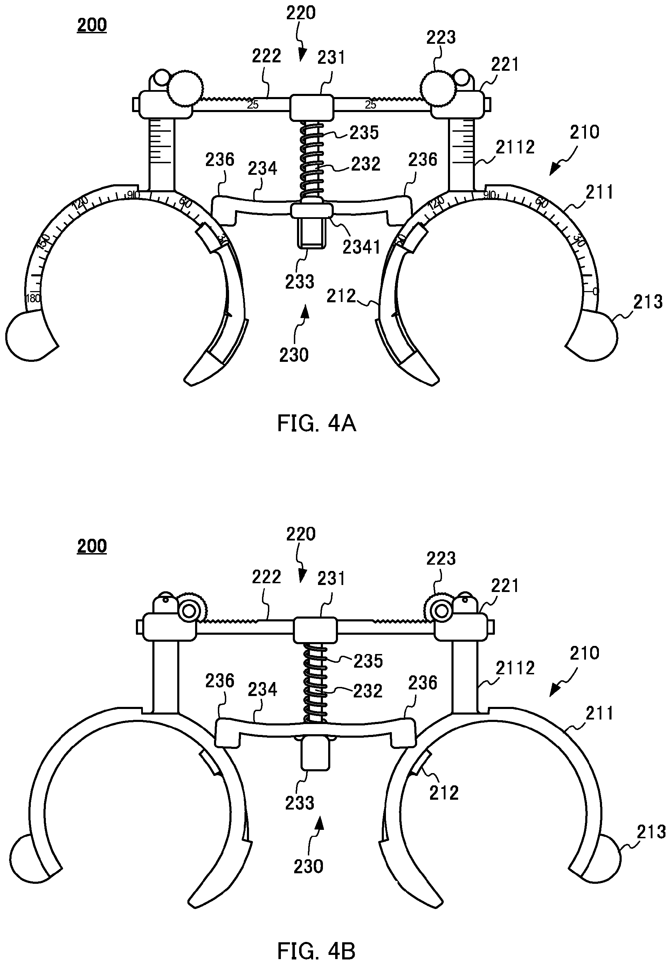

FIG. 4A and FIG. 4B are diagrams for explaining lens holder 200. FIG 4A illustrates a state of viewing lens holder 200 from the front side, FIG 4B illustrates a state of viewing lens holder 200 from the rear side, respectively. As lens holder 200, at least one lens unit 300 may be disposed on the object surface side of electronic lens 110 of electronic glasses 100, the position thereof may be adjusted. Although it is not particularly limited, a trial attachment manufactured by Hasegawa Bicoh Co., Ltd. can be used, for example.

As illustrated in FIGS. 4A and 4B, lens holder 200 has a pair of holders 210, position adjuster 220, and attaching portion 230. As illustrated in FIG. 1, lens holder 200 is used by attaching to electronic glasses 100 from the objective surface side of electronic lens 110 of electronic glasses 100.

Holder 210 holds lens unit 300 with a frame, wherein lens unit 300 is different from electronic lens 110 of electronic glasses 100 described above. More specifically, as illustrated in the drawing 4A, holder 210 includes outer frame portion 211, first supporting portion 212, and second supporting portion 213.

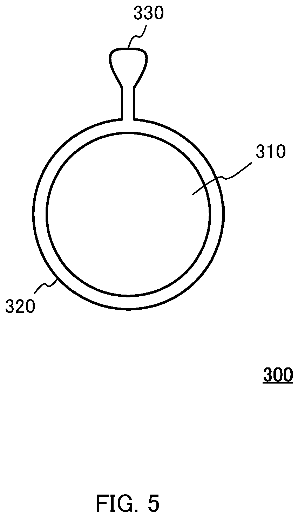

Lens unit 300 is a unit that a single lens, commonly referred to as an ophthalmoscopic lens or a trial lens or the like, is encased in a frame. FIG. 5 is a diagram for explaining lens unit 300. As illustrated in FIG. 5, lens unit 300 has lens 310, frame portion 320, and knob portion 330. Lens 310 is an example of the second lens of the present invention. In Embodiment 1, lens 310 of lens unit 300 is a normal lens that does not have a region in which optical characteristics change. Lens unit 300 does not need to be specially designed for eyewear system 1, and a commercially available ophthalmoscopic lens and/or the like may be used as appropriate. Note that the present invention is not limited to this, and lens unit 300 may have a region in which optical characteristics change in accordance with electrical control and/or the like. When adopting a lens which optical characteristics are changed by electrical control to lens unit 300, for example, the input unit for changing the optical characteristics of the lens to knob portion 330 may be provided.

Lens 310 is a various lens, such as a myopic lens, a hyperopic lens, an astigmatic lens, a color lens, a polarized lens, a light control lens, an antidazzle lens, a specific wavelength cut lens that reduce the transmission of specific wavelengths of light (e.g., lenses that reduce the transmission of light at wavelengths from 380 nm to around 420 nm, lenses that reduce the transmission of light at wavelengths from 460 nm to around 480 nm, lenses that reduce the transmission of light at wavelengths around 585 nm). Lens 310 is selected as appropriate for the purpose of the user.

Lens unit 300 is supported by first supporting portion 212 and second supporting portion 213 of holder 210, and is held in a state of being positioned relative to outer frame portion 211. The holding of lens unit 300 by holder 210 is performed by first supporting portion 212 and second supporting portion 213 coming into contact with the frame portion 320.

First supporting portion 212 and second supporting portion 213 is formed in a shape such that attachment and detachment of lens unit 300 is easy. Attachment of lens unit 300 to holder 210, for example, is performed by, for example, the user holding knob portion 330 and inserting frame portion 320 of lens unit 300 between first supporting portion 212 and second supporting portion 213. Removal of lens unit 300 from holder 210 is performed, for example, by the user pinching knob portion 330 and extracting lens unit 300 from between first supporting portion 212 and second supporting portion 213.

The shape of the inner peripheral surface of first supporting portion 212 and second supporting portion 213 may be formed so as to suitably support lens unit 300 and to be easily detachable, in accordance with the shape of the frame portion 320 of lens unit 300 held by holder 210. The inner peripheral surface of first supporting portion 212 or second supporting portion 213 means the surface of first supporting portion 212 or second supporting portion 213 in contact with the frame portion 320 in a state where lens unit 300 is held by holder 210. In the example illustrated in FIG. 4A, the inner peripheral surface of first supporting portion 212 is formed in an arc shape in accordance with the shape of the frame portion 320 of lens unit 300 of which outer periphery is circular as illustrated in FIG. 5. Although the shape of the inner peripheral surface of second supporting portion 213 is also formed in a similarly arc shape, it is formed short in the circumferential direction as compared with first supporting portion 212, in order to facilitate attachment and detachment of lens unit 300.

In the examples illustrated in FIG. 4A and FIG. 4B, first supporting portion 212 and second supporting portion 213 are formed so as to be able to support one lens unit 300. However, the present invention is not limited to this, first supporting portion 212 and second supporting portion 213 may be formed so as to be able to support a plurality of lens units 300. In this case, the plurality of lens units 300 supported by first supporting portion 212 and second supporting portion 213 are overlapped in the front-back direction and held in a state of being closely attached or close to each other. As a result, a plurality of lens units 300 having respectively different characteristics can be used in combination, depending on the purpose.

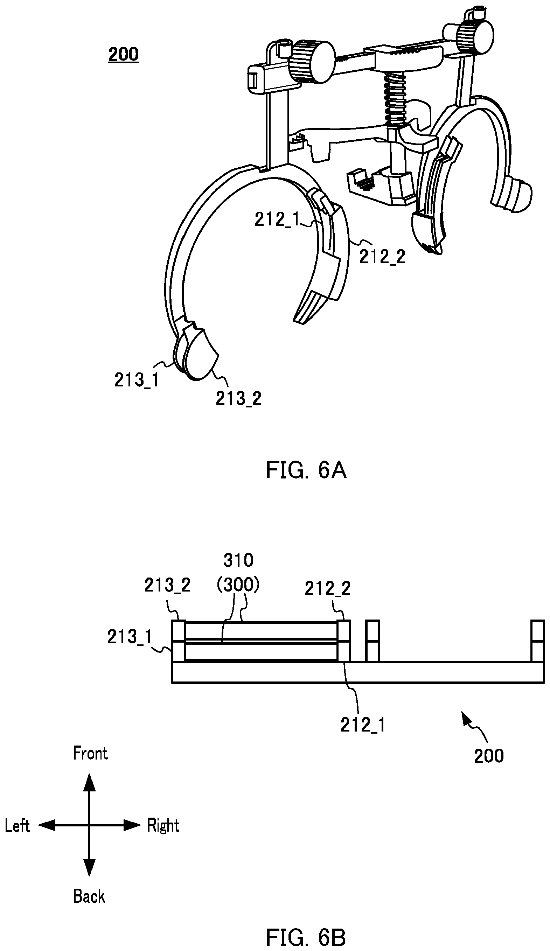

FIG. 6A is a perspective view illustrating lens holder 200 in the case that two sets of first supporting portion 212 and second supporting portion 213 are provided along the front-back direction. FIG. 6B is a diagram illustrating a condition in which two lens units 300 are inserted into lens holder 200 in which two sets of first supporting portion 212 and second supporting portion 213 are provided along the front-back direction. In FIG. 6B, lens holder 200 and lens unit 300 are viewed from above. In FIG. 6B, the scale of the left-right direction and the front-back direction is not accurate, each part is illustrated with emphasis particularly in the front-back direction.

As illustrated in FIGS. 6A and 6B, when two sets of first supporting portion 212 and second supporting portion 213 are provided, two lenses having mutually different characteristics, such as the myopic lens and the astigmatic lens, are overlapped in the front-back direction, and are held in a state being closely attached each other or close to each other. Examples of combinations of two lenses with different characteristics include, for example, a combination of the myopic lens or the hyperopic lens and the astigmatic lens, and a combination of the myopic lens or the hyperopic lens and the color lens, the polarized lens, the light control lens, the antidazzle lens, or the specific wavelength cut lens.

For example, three sets of first supporting portion 212 and second supporting portion 213 may be provided so as to support three lens units 300. In this case, three lenses having different characteristics can be held at the same time. In such cases, for example, in addition to the myopic lens or the hyperopic lens and the astigmatic lens, the color lens, the polarized lens, the light control lens, the antidazzle lens, or the specific wavelength cut lens may be overlapped in the front-back direction and may be held in a state being closely attached each other or close to each other.

Outer frame portion 211 may have a scale indicating the angle, so as to adjust the angle of lens unit 300, when lens unit 300 is supported on first supporting portion 212 and second supporting portion 213. In the example illustrated in FIG 4A, the scale as can be measured up to 180 degrees (0, 30, 60, 90, 120, 150, and 180) on the basis of the left side in the horizontal direction as a reference (0 degrees) is provided on outer frame portion 211. Such a scale is used to rotate lens 310 of astigmatic lens unit 300 about the central axis, in accordance with, for example, the state of astigmatism of the user. When first supporting portion 212 and second supporting portion 213 is formed so as to be able to support the two lens units 300, lens 310 of at least one lens unit 300 may be configured to be rotated about the central axis.

First supporting portion 212 and second supporting portion 213 are fixed to outer frame portion 211. Outer frame portion 211 has arm portion 2112. In the example illustrated in FIG 4A and FIG 4B, arm portion 2112 is a flat plate-shaped member, and is connected to position adjuster 220. The position of entire holder 210 is adjusted by being adjusted the position of arm portion 2112 by position adjuster 220. Note that the shape of arm portion 2112 may not be a flat plate, and may be, for example, a rod shape.

Position adjuster 220 is configured to adjust the position of holder 210. Position adjuster 220 is an example of the second lens position adjuster of the present invention. Position adjuster 220 includes arm holder 221, beam portion 222, and gear portion 223.

In the example illustrated in FIG 4A and FIG 4B, arm holder 221 is a cylindrical member to which plate-shaped arm portion 2112 is inserted, and holds arm portion 2112 extending in the up-down direction in a state movable in the up-down direction. The inner peripheral surface of arm holder 221 is formed in such a size as to be closely attached with the outer peripheral surface of arm portion 2112, and arm holder 221 holds arm portion 2112 by its frictional force. Alternatively, a plurality of convex portions may be provided on the inner peripheral surface of arm holder 221 and the outer peripheral surface of arm portion 2112 respectively, and arm holder 221 may hold arm portion 2112 more firmly by meshing the convex portion of arm holder 221 and the convex portion of arm portion 2112 with each other. In this case, a plurality of convex portions may be provided on at least one surface of the inner surface of arm holder 221, and on at least one surface of the outer peripheral surface of arm portion 2112 facing this surface. The movement of arm portion 2112 in the up-down direction relative to arm holder 221 may be performed by the user pinching and moving a portion of arm portion 2112 protruding from the upper side of arm holder 221. A scale may be marked on arm portion 2112 so that the amount of movement in the up-down direction can be known.

Arm holder 221 is movably connected to beam portion 222 in the left-right direction. Beam portion 222 is a member having, for example, a square bar shape, and extending in the left-right direction. In the present invention, beam portion 222 may not have a square bar shape, and may have a round bar shape, for example. For example, a plurality of convex portions are provided on the upper surface of beam portion 222, and some of the convex portions mesh with a portion of the gear of the gear portion 223 rotatably provided on arm holder 221. One end portion in the front-back direction of the gear portion 223 is formed in a disc shape so that it can be rotated by the user. The gear portion 223 connected integrally with the disc is rotated by the user rotating the disc, and arm portion holder 221 is moved in the left-right direction along beam portion 222 accordingly. A number indicating the length from the left and right ends may be provided on beam portion 222, so that the amount of movement in the left-right direction can be seen. In the example illustrated in FIG. 4A, the number "25" is provided in a position where the distance from the end is 25 mm on beam portion 222.

With such a configuration, the position of holder 210 can be adjusted in the vertical and left-right directions relative to beam portion 222 by position adjuster 220. Although one of a pair of holders 210 has been described above, the other holder 210 has the same configuration. Note that the other holder 210 is configured bilaterally symmetrically with one of holder 210.

Attaching portion 230 for attaching lens holder 200 to electronic glasses 100 is connected in the central portion of beam portion 222. Attaching portion 230 can be attached to lens holder 200 so that the position of beam portion 222 is uniquely determined relative to electronic glasses 100. Thus, the positions of a pair of holders 210 in the vertical and the left-right directions can be suitably adjusted relative to electronic glasses 100 by position adjuster 220 described above.

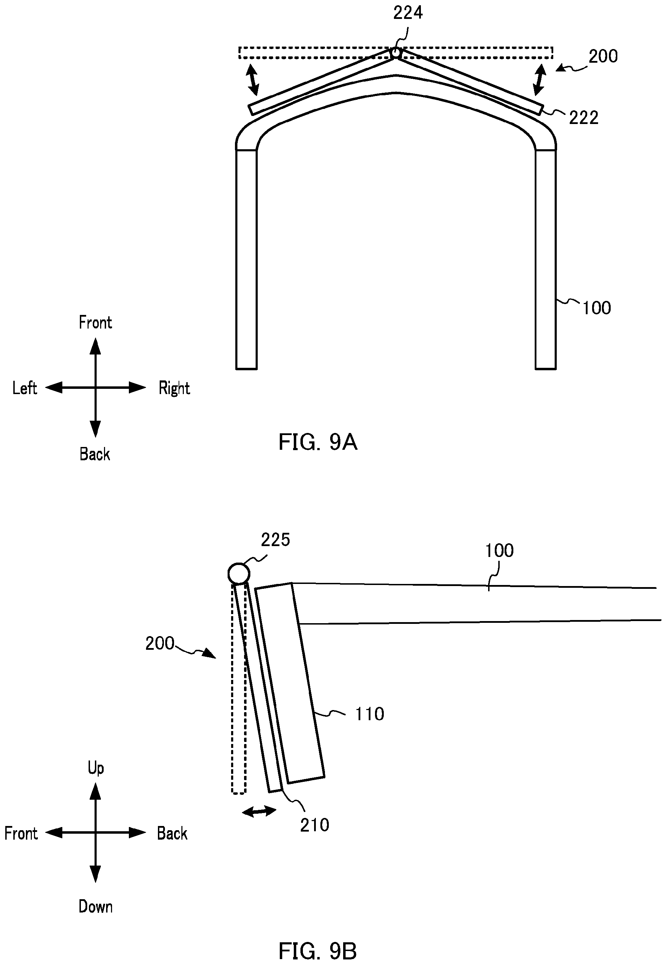

Although not illustrated in FIG. 4A and FIG. 4B, position adjuster 220 may have first movable portion 224 (see FIG. 9A) described later. First movable portion 224 is an example of the curve adjuster of the present invention. First movable portion 224 is provided, for example, in the vicinity of the center of beam portion 222, and includes a movable shaft nearly along the up-down direction of electronic glasses 100. Specifically, first movable portion 224 is, for example, a hinge. Beam portions 222 on both sides of first movable portion 224 can be adjusted to a desired angle with first movable portion 224 as a rotation center (movable center). In other words, first movable portion 224 is configured to yaw the left and right beam portions 222 and holder 210 fixed to beam portion 222. First movable portion 224 is not limited to a hinge, may be any adjustable configuration in accordance with the curve of front 130 of electronic glasses 100, and may have a bellows structure, for example. With the configuration of first movable portion 224 described above, when front 130 of electronic glasses 100 is curved, the angle of the lens surface of lens 310 (second lens) of lens unit 300 to be held in holder 210 can be adjusted in accordance with the curve of front 130 of electronic glasses 100, so as to suitably face the lens surface of the electronic lens 110 (first lens) of electronic glasses 100.

Although not illustrated in FIG. 4A and FIG. 4B, position adjuster 220 may have second movable portion 225 (see FIG. 9B) described later. Second movable portion 225 is an example of the inclination adjuster of the present invention. Second movable portion 225 is provided, for example, in the vicinity of the connecting portion between beam portion 222 and attaching portion 230 (connecting portion 231 described later), and includes a movable shaft along nearly the left-right direction of electronic glasses 100. Specifically, second movable portion 225 is, for example, a hinge. Entire beam portions 222 can be adjusted to a desired angle with second movable portion 225 as a rotation center (movable center). In other words, second movable portion 225 is configured to pitch beam portion 222 and entire holder 210. Second movable portion 225 is not limited to a hinge, may be any adjustable configuration in accordance with the inclination of electronic lens 110 of electronic glasses 100, and may have a bellows structure for example. With the configuration of second movable portion 225 described above, the angle of the lens surface of lens 310 (second lens) of lens unit 300 to be held in holder 210 can be adjusted in accordance with the inclination of front 130 of electronic glasses 100, so as to suitably face the lens surface of the electronic lens 110 (first lens) of electronic glasses 100.

Position adjuster 220 may have both first movable portion 224 and second movable portion 225. In that case, first movable portion 224 and second movable portion 225 are configured to cooperate and to function as an angle adjuster for adjusting the angle of the lens surface of lens 310 of lens unit 300, so that the lens surface of lens 310 (second lens) of lens unit 300 faces horizontally to the lens surface of electronic lens 110 (first lens) of electronic glasses 100.

Attaching portion 230 has connecting portion 231, shaft member 232, upward claw portion 233, attaching beam portion 234, spring 235, and a pair of downward claw portion 236. Attaching portion 230 is an example of the second attaching portion of the present invention.

Connecting portion 231 is a member for connecting the central portion of the upper end and beam portion 222 of shaft member 232. Shaft member 232 is a rod-shaped member extending in the up-down direction. At the lower end of shaft member 232, upward claw portion 233 is provided.

Between connecting portion 231 and upward claw portion 233 in shaft member 232, attaching beam portion 234 extending nearly in the left-right direction is provided to be movable in the up-down direction. More particularly, shaft member 232 is fitted into the hole (not illustrated) provided in the central portion of attaching beam portion 234, the diameter of the hole is formed slightly larger than the diameter of shaft member 232, so that attaching beam portion 234 can be moved in the up-down direction relative to shaft member 232. At the central portion of attaching beam portion 234, gripping portion 2341 for the user gripping at attaching is provided so as to protrude, for example, toward the front.

Between connecting portion 231 and attaching beam portion 234, spring 235 is provided so as to surround shaft member 232. Spring 235 urges the attaching beam portion 234 downwardly relative to the connection 231. At the left and right ends of attaching beam portion 234, a pair of downward claw portion 236 is provided.

Using such an attaching portion 230, a method of attaching lens holder 200 to electronic glasses 100 is as follows, for example. When the user pushes attaching beam portion 234 upward by gripping the gripping portion 2341, attaching beam portion 234 and a pair of downward claw portion 236 is moved upward, a gap is generated between a pair of downward claw portion 236 and upward claw portion 233. When front 130 of electronic glasses 100 is inserted into this gap, and the user reduces the force to grip gripping portion 2341, downward force is applied to attaching beam portion 234 and a pair of downward claw portion 236 by the biasing force of spring 235. Thus, front 130 of electronic glasses 100 is sandwiched between upward claw portion 233 and a pair of downward claw portion 236. Thus, since lens holder 200 is supported at three points with respect to frame 120 of electronic glasses 100, lens holder 200 is attached so as not to move relative to electronic glasses 100.

In Embodiment 1, attaching portion 230 of the type in which front 130 of electronic glasses 100 is sandwiched between upward claw portion 233 and a pair of downward claw portions 236 has been described. However, the present invention is not limited thereto. For example, a clip-shaped member biased in a closing direction by a spring may be adopted as attaching portion 230. In this case, it is desirable that the clip-shaped member is covered with a soft material such as resin so that electronic glasses 100 are not damaged by the clip-shaped member.

### [Usage of Eyewear System 1]

The usage of eyewear system 1 will be described in detail below. As described above, eyewear system 1 is used by attaching lens holder 200 to electronic glasses 100. Eyewear system 1 is mainly used for the purpose of allowing the user to experience the difference in the viewing way due to the change in optical characteristics in first region 111 of electronic glasses 100. In the following description, the experiencing user is referred to as an experiencer.

Since the experiencer who experiences eyewear system 1 is a user who intends to use electronic glasses 100, the experiencer has often weaker vision than usual. Meanwhile, electronic lens 110 of electronic glasses 100 for experiencing does not have a diopter or is suited to a predetermined diopter (including a progressive design) and is not suited to the visual acuity of the experiencer. For this reason, when the experiencer with weak vision wears electronic glasses 100 for experiencing, the experiencer sees the surroundings through a lens not suited to the experiencer's own vision. In many cases, the experiencer cannot obtain a good field of view even through electronic glasses 100. Even if the optical characteristics of first region 111, which is a part of electronic lens 110, change in this state, it is difficult for the experiencer to recognize the difference in the viewing way in first region 111.

Therefore, in eyewear system 1 according to Embodiment 1, a clear field of view is given to the experiencer by attaching lens unit 300 having a diopter suited to the visual acuity of the experiencer to lens holder 200. In this state, the experiencer can suitably experience the difference in the viewing way due to the change in the optical characteristics in first region 111.

When the experiencer experiences electronic glasses 100 using eyewear system 1, first, the experiencer wears electronic glasses 100 to which lens holder 200 is attached.

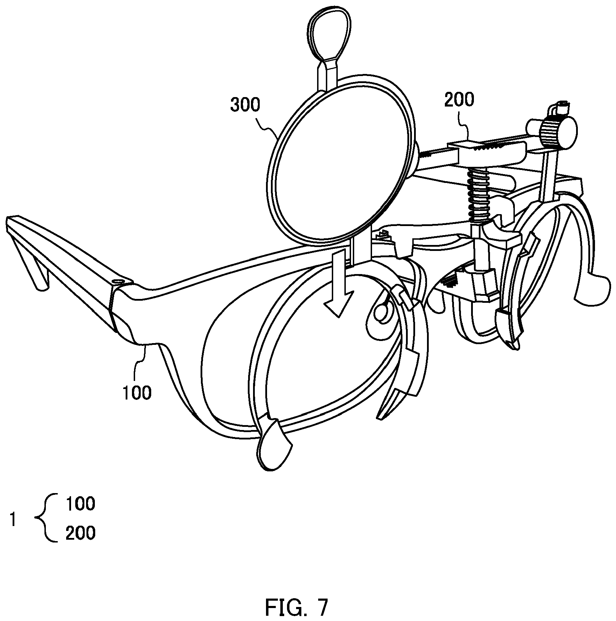

Next, as illustrated in FIG. 7, lens unit 300 suited to the visual acuity of the experiencer is attached to lens holder 200. FIG. 7 is a diagram illustrating a state in which lens unit 300 suited to the visual acuity of the experiencer is attached to lens holder 200. Lens unit 300 suited to the visual acuity of the experiencer is, for example when the experiencer is myopic, a myopic lens having the diopter suited to the visual acuity. Alternatively, when the experiencer is hyperopic, lens unit 300 is a hyperopic lens having the diopter suited to the visual acuity. If the experiencer is astigmatic, lens unit 300 with the astigmatic lens is attached to lens holder 200 at an angle suited to the axial angle of the subject's astigmatism. The diopter of the myopic or hyperopic lens may be determined, for example, when the experiencer has already used the glasses, in accordance with the diopter of the lens used in the glasses.

When lens holder 200 can hold a plurality of lenses, a plurality of lenses can be combined. If the experiencer is myopic or hyperopic and astigmatic at the same time, lens unit 300 having the myopic lens or the hyperopic lens together with lens unit 300 having the astigmatic lens are attached to lens holder 200 at the same time. In response to the experient's request, a plurality of lens unit 300 with the myopic lens, the hyperopic lens, the astigmatic lens, the color lens, the polarized lens, the light control lens, the antidazzle lens, or the specific wavelength cut lens and/or the like may be appropriately combined and may be attached to lens holder 200.

The attachment of lens unit 300 to lens holder 200 is desirably performed by, for example, a shop clerk and/or the like in a shop where experiences eyewear system 1 to the experiencer. However, the experiencer may perform the attachment by oneself.

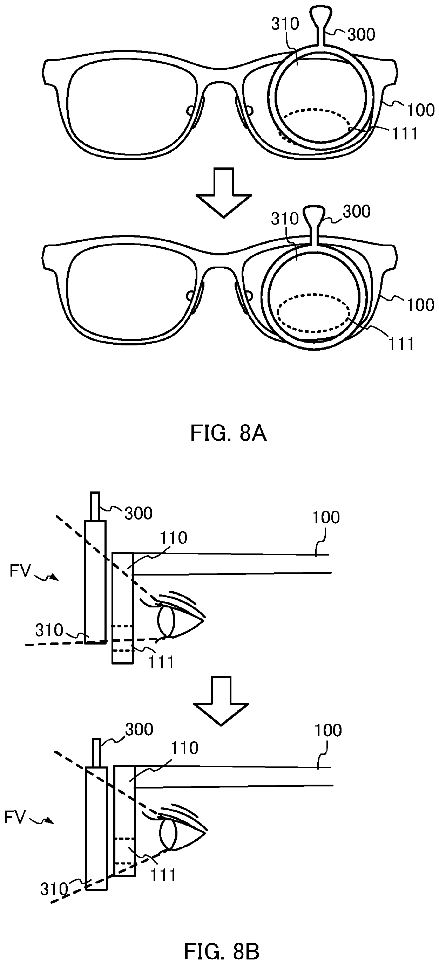

Next, as illustrated in FIG. 8A and FIG. 8B, the position of lens unit 300 is adjusted. FIG. 8A is a diagram illustrating a state of positional adjustment of lens unit 300 as viewed from the front side. FIG. 8B is a diagram illustrating a relation between the positional adjustment of lens unit 300 and the field of view of the experiencer as viewed from the lateral side. In FIG. 8A and FIG. 8B, the upper side of the arrow illustrates the state before the position adjustment, the lower side of the arrow illustrates the state after the position adjustment, respectively. In FIG. 8B, the scale of the left-right direction and the front-back direction is not accurate, it is illustrated with emphasis on each portion in the front-back direction in particular. FIG. 8A and FIG. 8B illustrate the positional relationship between lens 310 of lens unit 300 and first region 111 of electronic glasses 100, illustration of lens holder 200 is omitted.

The positional adjustment of lens unit 300 is desirably performed by the shop clerk and/or the like. However, it may be performed by the experiencer oneself. The positional adjustment of lens unit 300 is performed such that first region 111 is positioned within the experient's field of view through lens 310 of lens unit 300. Specifically, as illustrated in Fig. 8B, when first region 111 protrudes from the experient's field of view FV through the lens 310, lens unit 300 is moved toward the protruding direction. As a result, first region 111 can be positioned in the experient's field of view FV through lens 310. The fine adjustment of the position of lens unit 300 may be performed appropriately so that first region 111 is located at a more suitable position for the experiencer in the experient's field of view FV through lens 310.

According to the procedure described above, first region 111 can be positioned in the field of view suited to the visual acuity of the experiencer. By operating input portion 143 of electronic glasses 100 in this state, the experiencer can suitably experience the difference in the viewing way due to the change in optical characteristics in first region 111. It is more desirable that the operation of input portion 143 is performed by the experiencer, not by the shop clerk.

When position adjuster 220 of lens holder 200 has first movable portion 224, as illustrated in FIG. 9A, even if front 130 of electronic glasses 100 is curved, the position of lens 310 held in holder 210 can be adjusted in accordance with the curve. When position adjuster 220 of lens holder 200 has second movable portion 225, as illustrated in FIG. 9B, even if electronic lens 110 of electronic glasses 100 is inclined in the front-back direction, the inclination of lens 310 can be adjusted in accordance with the inclination of electronic lens 110. With such a configuration, the experiencer can more suitably experience the difference in the viewing way due to the change in optical characteristics in first region 111.

### <Effects>

As described above, eyewear system 1 according to Embodiment 1 includes electron glasses 100 (eyewear) having electron lens 110 (the first lens) including first region 111 (the optical characteristic change region) in which the optical characteristics change, and frame 120 for holding electron lens 110, and lens holder 200 having holder 210 holding lens unit 300 so that lens 310 (the second lens) of lens unit 300 different from electronic lens 110 face to one surface of the electron lens 110 and first region 111 is positioned in the field of view through lens 310 of the experiencer (the user) and attaching portion 230 for attaching to the frame 120.

In such a configuration, by attaching lens unit 300 suited to the visual acuity of the experiencer to lens holder 200, first region 111 can be positioned in the experient's field of view through lens 310 suited to the visual acuity. By operating input portion 143 of electronic glasses 100 in this state, the experiencer can suitably experience the difference in the viewing way due to the change in the optical characteristics of first region 111.

In eyewear system 1 according to Embodiment 1, lens retainer 200 has position adjuster 220 that adjusts the relative position of the lens unit 300 to the electron lens 110. With such a configuration, when first region 111 is not positioned in the field of view suited to the visual acuity of the user, first region 111 can be positioned in the field of view suited to the experient's visual acuity by adjusting the position of lens unit 300 by position adjuster 220. The position of lens unit 300 can be appropriately adjusted so that the position of lens 310 becomes a suitable position for the experiencer.

### (Embodiment 2)

In Embodiment 1 described above, it have been described about the eyewear system giving the experiencer the experience of the difference in the viewing way due to the change in the optical characteristics of electronic lens by attaching the lens holder for holding the lens unit of the predetermined diopter to the electronic glasses having the electronic lens. In Embodiment 2 described below, it will be described about an eyewear system giving the experiencer an experience of a difference in the viewing way due to a change in the optical characteristics of an electronic lens by attaching an overglass having an electronic lens to the experient's glasses. Note that, in Embodiment 2, the overglass means an eyewear that is attached so as to cover the other eyewear.

In the following description, in order to avoid complication, the glasses possessed by the experiencer themselves are referred to as experient's glasses.

### [Configuration of Overglass 400]

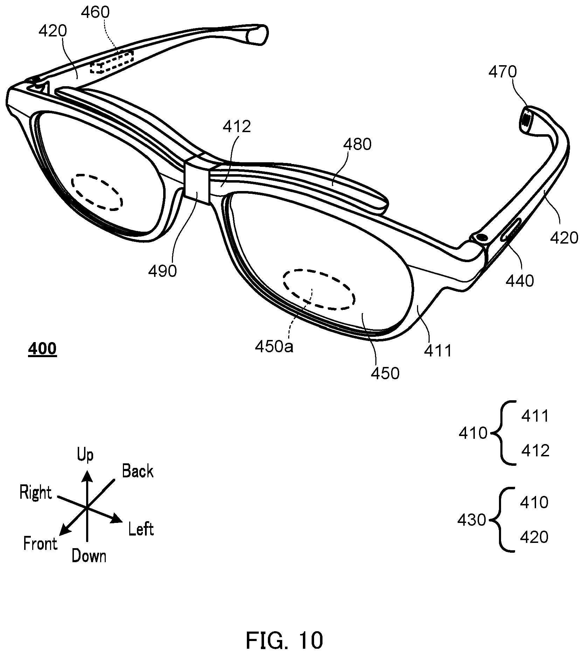

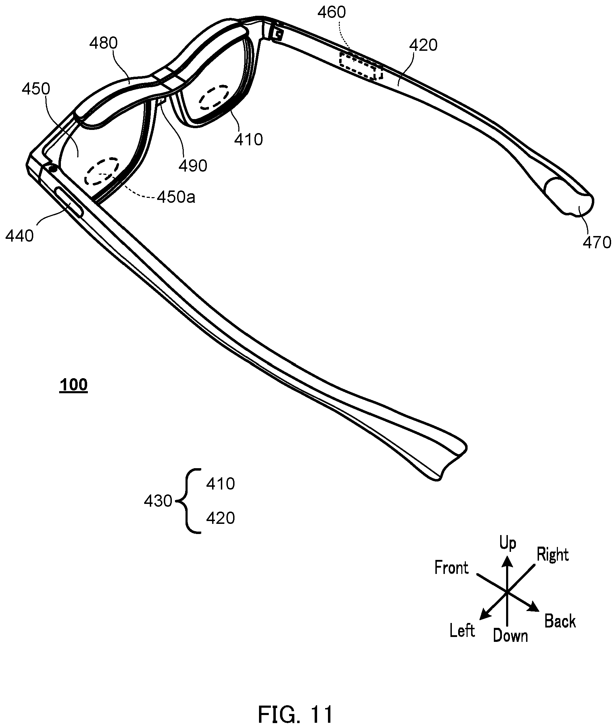

FIG. 10 and FIG. 11 are diagrams illustrating the appearance of overglass 400 according to Embodiment 2. Overglass 400 is an example of the first frame of the present invention. FIG. 10 is a perspective view from the front of overglass 400, and FIG. 11 is a perspective view from the rear of overglass 400. In Embodiment 2, the front-back direction, the up-down direction, and the left-right direction of overglass 400 correspond to the front-back direction, the up-down direction, and the left-right direction relative to the face of the experiencer wearing overglass 400, respectively. Specifically, the X-axis direction in each drawing is the front-back direction (the direction indicated by the arrow is forward), the Y-axis direction is the left-right direction (the direction indicated by the arrow is leftward), and the Z-axis direction is the up-down direction (the direction indicated by the arrow is upward).

As illustrated in FIG. 10 and FIG. 11, overglass 400 has frame 430, input portion 440, electronic lens 450, control unit 460, power source 470, attaching portion 480, and position adjuster 490. Attaching portion 480 is an example of the first attaching portion of the present invention. Position adjuster 490 is an example of the first lens position adjuster of the present invention.

Frame 430 has front 410 and a pair of temples 420.

Front 410 holds a pair of electronic lenses 450. Each of a pair of electronic lenses 450 corresponds to the left and right eyes of the experiencer respectively. Front 410 has a pair of rims 411 for supporting a pair of electronic lenses 450, respectively, and bridge 412 for connecting a pair of rims 411. The shape of rim 411 corresponds to the shape of electronic lens 450. Although not illustrated in particular, the interior of front 410 (between rim 411 and electronic lens 450), wiring for electrically connecting electronic lens 450 and control unit 460 is disposed.

Front 410 also has attaching portion 480 for attaching overglass 400 to the experient's glasses when overglass 400 is used with the experient's glasses. As illustrated in FIG. 10 and FIG. 11, attaching portion 480 is a member disposed in the vicinity of a nearly central portion of front 410 (i.e., a central portion in the left-right direction of overglass 400) along the shape of front 410 at the top of front 410. However, attaching portion 480 does not need to be connected to front 410 in its entirety, and only a part thereof may be connected to front 410. In FIG. 10 and FIG. 11, an example that attaching portion 480 is connected to front 410 only in the vicinity of bridge 412 is illustrated.

Front 410 has position adjuster 490 for adjusting the position of electronic lens 450 relative to the lens of the experient's glasses when overglass 400 is used with the experient's glasses. As illustrated in FIG. 10 and FIG. 11, position adjuster 490 is disposed at a position where attaching portion 480 is connected to front 410. Details of attaching portion 480 and position adjuster 490 will be described later.

The material of front 410 is not particularly limited, and a known material which is generally used as a material of the front of the glasses can be adopted. The material of front 410 may be appropriately selected from, for example, metals such as titanium, aluminum, and stainless steel, resins such as polyamide, acetate, celluloid, polyetherimide and polyurethane, or carbon and/or the like.

A pair of temples 420 are a pair of rod-shaped members disposed so as to be nearly bilaterally symmetrical, and are connected to front 410 at the front end thereof. Input portion 440 is disposed on one or both of a pair of temples 420. Control unit 460 is disposed on one of a pair of temples 420. At one or both back end of a pair of temples 420 (the end farther from front 410), power source 470 is disposed. Although not illustrated in particular, inside temple 420, input portion 440, control unit 460, and wiring for electrically connecting power source 470 are disposed.

The material of temple 420 is not particularly limited, and may be a known material used as a material of a temple of glasses. The material of temple 420 is appropriately selected from, for example, metals such as titanium, aluminum and stainless steel, resins such as polyamide, acetate, celluloid, polyetherimide and polyurethane, or carbon and/or the like.

In the present embodiment, frame 430 including front 410 and temple 420, and electronic lens 450 are the members constituting the appearance of overglass 400. Frame 430 including front 410 and temple 420, and electric lens 450 are formed and arranged so as to be nearly bilaterally symmetrical on the basis of the center of frame 430.

Input portion 440 receives an input operation from the experiencer and/or the like wearing overglass 400. Specifically, input portion 440 is a plurality of capacitive touch sensors disposed in the outer and front regions of temple 420. Input portion 440 may be a sensor other than the touch sensor, and the number of sensor devices may be one or more.

A pair of electronic lenses 450 are lenses having liquid crystal lenses 450a whose optical characteristics are changed by application of a voltage, and held by front 410 of frame 430. Electronic lens 450 may be a spherical lens or an aspherical lens.

Electronic lens 450 has a multilayer structure in which a plurality of layers overlap in the thickness direction. A part of the multilayer structure is liquid crystal lens 450a having a liquid crystal layer (not illustrated) sandwiched between a pair of conductive layers (not illustrated). As illustrated in FIG. 10 and FIG. 11, liquid crystal lens 450a occupies a part of an area of electronic lens 450. Transparent electrodes (not illustrated) are connected to a pair of conductive layers, and liquid crystal lens 450a is electrically connected to control unit 460 via the transparent electrodes. Liquid crystal lens 450a can change its refractive index when a voltage is applied between a pair of conductive layers under the control of control unit 460 according to an input operation to input portion 440. Liquid crystal lens 450a is an example of the electric element and the optical element of the present invention.

Control unit 460 controls the operation or non-operation of liquid crystal lens 450a in accordance with the input operation received by input portion 440. Control unit 460 is an arithmetic unit including, for example, CPU (Central Processing Unit), RAM (Random Access Memory), and ROM (Read Only Memory) and/or the like. Control unit 460 reads a program for executing the function of liquid crystal lens 450a from the ROM, expands the program in the RAM, and controls the operation of liquid crystal lens 450a executing the expanded program.

### [Details of Attaching portion 480 and Position Adjuster 490]

Hereinafter, attaching portion 480 and position adjuster 490 of front 410 of frame 430 has will be described in detail.

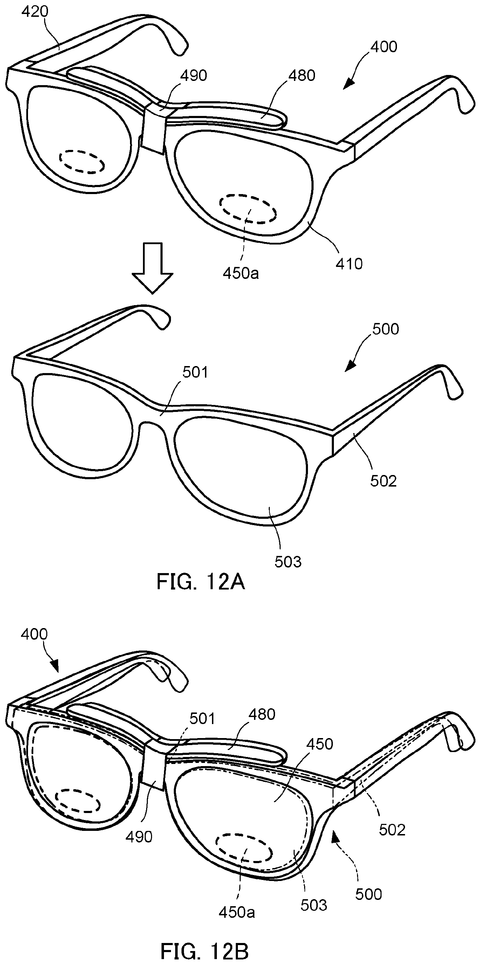

FIG. 12A and FIG. 12B are diagrams illustrating a state that overglass 400 is used with experient's glasses 500. Experient's glasses 500 have, for example, lenses having a diopter suited to the visual acuity of the experiencer who is trying to experience electronic lens 450 of overglass 400. Experient's glasses 500 are an example of the second frame of the present invention.

FIG. 12A is a perspective view illustrating a state before overglass 400 is attached to experient's glasses 500, and FIG. 12B is a perspective view illustrating a state after overglass 400 is attached to experient's glasses 500.

In the present embodiment, the attachment of overglass 400 to experient's glasses 500 means that overglass 400 is held by attaching portion 480 of overglass 400 coming into contact with front 501 of experient's glasses 500 from above, as illustrated in FIG. 12A. In the present embodiment, the state in which overglass 400 is attached to experient's glasses 500 is a state in which overglass 400 is placed on front 501 of experient's glasses 500 by attaching portion 480. Front 501 is an example of the second front frame of the present invention.

A portion other than attaching portion 480 of overglass 400 may or may not be in contact with experient's glasses 500. From the viewpoint of dispersing the weight of overglass 400, it is preferable that, for example, a portion of temple 420 of overglass 400 is in contact with temple 502 of experient's glasses 500. Temple 502 is an example of the second temple of the present invention.

The state in which overglass 400 is attached to experient's glasses 500 may be a state in which overglass 400 is fixed to experient's glasses 500, or may be a state in which it is not fixed. In Embodiment 2, the fixing means making overglass 400 a state not being easily detached from experient's glasses 500 by, for example, clipping, screwing, and/or the like. It is more preferable that overglass 400 is not fixed to experient's glasses 500, because, for example, various overglass 400 having different addition (differences in diopter from other portions of electronic lens 450) of liquid crystal lens 450a can be easily replaced to allow the experiencer to experience.

As illustrated in FIG. 12B, in a state in which overglass 400 is attached to experient's glasses 500, electronic lens 450 of overglass 400 and lens 503 of experient's glasses 500 overlap with each other. Thus, the experiencer can experience the field of view through both lens 503 of experient's glasses 500 and liquid crystal lens 450a of overglass 400. Therefore, even the experiencer with weak vision can suitably experience the change in the viewing way of overglass 400 by liquid crystal lens 450a. However, depending on the shapes and sizes of electronic lens 450 of overglass 400 and lens 503 of experient's glasses 500, liquid crystal lens 450a may not be in a suitable position as described later. In order to avoid such a situation, overglass 400 has position adjuster 490.

As illustrated in FIG. 10 to FIG. 12B, position adjuster 490 for adjusting the position of electronic lens 450 relative to experient's glasses 500 is provided at front 410 of overglass 400. Position adjuster 490 enables position adjustment of electronic lens 450 relative to lens 503 of experient's glasses 500. Although the method of realizing the position adjustment of position adjuster 490 is not particularly limited in the present invention, as an example, the position adjustment may be performed by the following method, for example. Position adjuster 490 has a mechanism for adjusting the position of, for example, a screw or rail, and may be configured to adjust the position of electronic lens 450 relative to attaching portion 480 by the rotation of the screw. With such a configuration, the position of electronic lens 450 relative to lens 503 of experient's glasses 500 can be adjusted. In the present embodiment, position adjuster 490 is disposed nearly at the center of overglass 400 in the left-right direction.

In the present embodiment, position adjuster 490 can perform position adjustment in three directions. The three directions are, i.e., the front-back direction, the left-right direction, and the up-down direction. The front-back direction is an example of a direction in which electronic lens 450 approaches or moves away from lens 503 of experient's glasses 500 (the first direction of the present invention). The left-right direction and the up-down direction are examples of directions perpendicular to the first direction (the second direction of the present invention).

### [Detailed Description of Position Adjustment]

Hereinafter, the position adjustment of electronic lens 450 by position adjuster 490 will be described in detail. In the following description of the position adjustment, it is assumed that overglass 400 has the bifocal lens. That is, the description will be made on the assumption that liquid crystal lens 450a is used for a short distance, and portions of electronic lens 450 other than liquid crystal lens 450a are used for a medium or long distance.

### <Front-back direction>

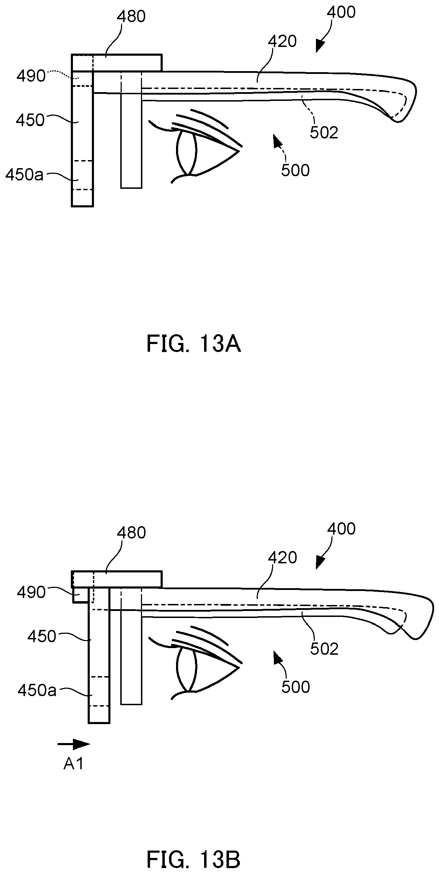

Referring to FIG. 13A and FIG. 13B, the position adjustment in the front-back direction will be described. Generally, in the front-back direction, the position of lens 503 of experient's glasses 500 is considered to be a suitable lens position for the experiencer. Therefore, it is desirable that the position adjustment in the front-back direction in a state in which overglass 400 is attached to experient's glasses 500 is performed in a direction of bringing electronic lens 450 of overglass 400 as close as possible to lens 503. FIG. 13A is a diagram illustrating a state before the position adjustment in the front-back direction is performed after overglass 400 is attached to experient's glasses 500. FIG. 13B is a diagram illustrating a state in which the position adjustment is performed so that electronic lens 450 approaches the lens of experient's glasses 500 in the front-back direction of overglass 400 in a state in which overglass 400 is attached to experient's glasses 500. Arrow A1 illustrated in FIG. 13B exemplifies the movement direction of electronic lens 450 in the front-back direction by position adjuster 490.

The position adjustment in the front-back direction is not limited to the adjustment in the direction of bringing electronic lens 450 close to lens 503, in some cases the adjustment in the direction of bringing electronic lens 450 away from lens 503 may be performed.

### <Left-right direction>

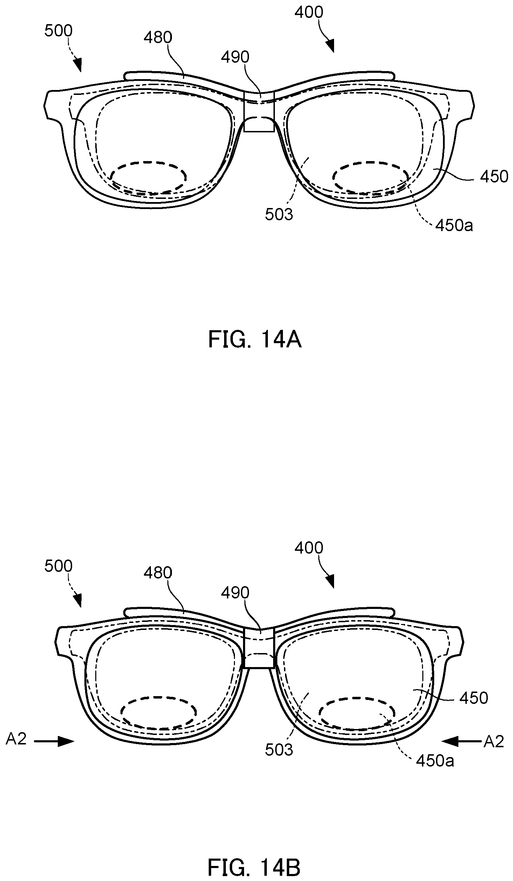

Next, referring to FIG. 14A and FIG. 14B, the position adjustment in the left-right direction will be described. FIG. 14A is a diagram illustrating a state before the position adjustment in the left-right direction is performed after overglass 400 is attached to experient's glasses 500. FIG. 14B is a diagram illustrating a state in which the position of overglass 400 is adjusted in the left-right direction in a state in which overglass 400 is attached to experient's glasses 500.

Generally, human eyes are closer when viewing close distances than when viewing medium or long distance. For this reason, it is necessary to make the eye points (the position where the line of sight passes through the lens) different between for short distance and for medium or long distance in the left-right direction, regarding the bifocal eyewear. Specifically, in the left-right direction, the eye point for short distance (near eye point) is positioned inside the eye point for medium or long distance (far eye point). The amount of deviation between the near eye point and the far eye point in the left-right direction is called inset.

From this viewpoint, in order for the experiencer experiencing suitably the change in the viewing way of overglass 400 by electronic lens 450, it is desirable that the position of liquid crystal lens 450a is adjusted so as to suit to the near eye point of the experiencer in the left-right direction. Arrow A2 in FIG. 14B exemplifies the movement direction of electronic lens 450 in the left-right direction by position adjuster 490.

In the present embodiment, the position adjustment in the left-right direction means the position adjustment in the direction that the left and right electronic lenses 450 approach or move away from position adjuster 490 disposed substantially in the center in the left-right direction of overglass 400. The position adjustment in the left-right direction by position adjuster 490 is performed nearly bilaterally symmetrically in the basis of position adjuster 490. Thus, it is possible to easily perform the position adjustment of a pair of electronic lenses 450 in the left-right direction.

### <Up-down direction>

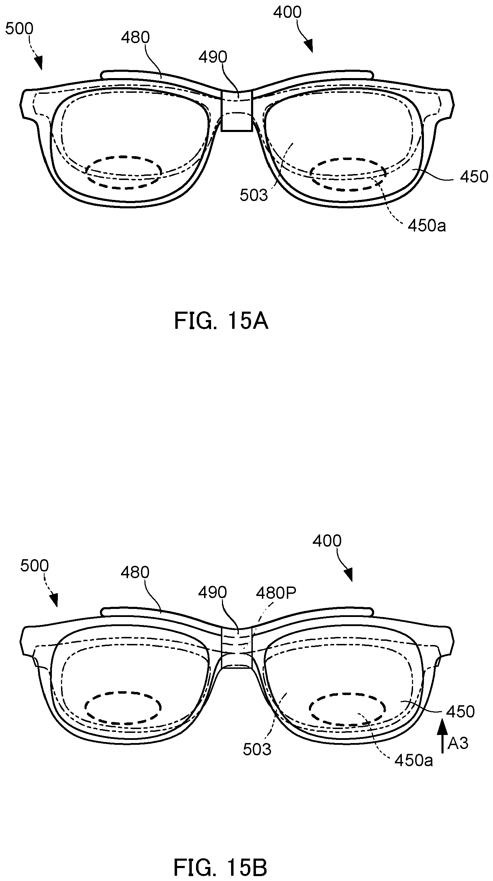

Referring to FIG. 15A and FIG. 15B, the position adjustment in the up-down direction will be described. FIG. 15A is a diagram illustrating a state before the position adjustment in the up-down direction is performed after overglass 400 is attached to experient's glasses 500. FIG. 15B is a diagram illustrating a state in which the position adjustment is performed in the up-down direction of overglass 400 in a state in which overglass 400 is attached to experient's glasses 500.

As illustrated in FIG. 15A, when the position adjustment is not performed by position adjuster 490, liquid crystal lens 450a may be in a state of protruding from lens 503 when viewed by the experiencer. Such a state may occur when the vertical length of electronic lens 450 and the vertical length of lens 503 are different each other. In such a case, since liquid crystal lens 450a is not located in the field of view of the experiencer through lens 503, it is difficult for the experiencer experiencing suitably the change in the viewing way by electronic lens 450 of overglass 400.

Therefore, it is desirable that the position adjustment by position adjuster 490 is performed so that the entire liquid crystal lens 450a is positioned within the field of view of the experiencer through lens 503 in the up-down direction.

More preferably, the position adjustment by position adjuster 490 is performed so that liquid crystal lens 450a is positioned below in the field of view of the experiencer through lens 503 in the up-down direction. The reason is that, in general bifocal glasses and/or the like, the lens for short distance is disposed below, the other regions are often used for medium or long distance.

FIG. 15B illustrates a state in which the position of electronic lens 450 in the up-down direction is adjusted so that liquid crystal lens 450a does not protrude from lens 503 when viewed by the experiencer and is disposed below lens 503. FIG. 15B illustrates a state that portions other than portion 480P of the attachment unit 480 is separated upward from experient's glasses 500 by the position adjustment in the up-down direction by position adjuster 490. Portion 480P of attaching portion 480 is a portion of attaching portion 480 that is not moved by position adjuster 490. In this case, the attachment of overglass 400 to experient's glasses 500 is performed by only portion 480P of the attachment part 480 coming into contact with experient's glasses 500. Arrow A3 illustrated in FIG. 15B exemplifies the movement direction in the up-down direction of electronic lens 450 by position adjuster 490.

In the above description related to FIG. 13A to FIG. 15B, it refers only to the position adjustment for electronic lens 450. In practice, position adjuster 490 of overglass 400 moves electronic lens 450 as well as other components of overglass 400 (frame 430 including front 410 and temple 420 and/or the like). As a result, the experiencer of overglass 400 can suitably fit overglass 400 to the shape of the head of the experiencer by using position adjuster 490.

### <Effects>

As described above, overglass 400 according to the embodiment of the present invention is an eyewear used together with experient's glasses 500, and includes electronic lens 450 having liquid crystal lens 450a, frame 430 holding electronic lens 450, attaching portion 480 attaching frame 430 to experient's glasses 500, and position adjuster 490 adjusting the relative position of electronic lens 450 to lens 503 of experient's glasses 500 in a state attached to experient's glasses 500.

With such a configuration, the position of electronic lens 450 relative to lens 503 of experient's glasses 500 can be suitably adjusted. Therefore, when overglass 400 of the present invention is used with experient's glasses 500, the position of liquid crystal lens 450a of electronic lens 450 can be set to a suitable position in the field of view of the experiencer. As a result, for example, the eyewear having the electrically controlled lens can allow the experiencer using the prescription glasses to suitably experience.

According to overglass 400 of the embodiment of the present invention, position adjuster 490 adjusts the relative position of experient's glasses 500 in the first direction approaching or moving away from lens 503 and/or in the second direction orthogonal to the first direction. The first direction corresponds to, for example, the front-back direction relative to the face of the experiencer wearing overglass 400, and the second direction corresponds to the left-right direction or the up-down direction. With such a configuration, the position of the electrically controlled lens can be easily adjusted to the suitable position.

In addition, according to overglass 400 of the embodiment of the present invention, position adjuster 490 moves the other components of overglass 400 together with electronic lens 450 when adjusting the position of electronic lens 450. Other configurations of overglass 400 refer to frame 430 including front 410 and temple 420 and/or the like. With such a configuration, position adjuster 490 can suitably fit overglass 400 to the shape of the head of the experiencer.

### [Variation]

While each of the embodiments of the present invention has been described above with reference to the accompanying drawings, the present invention is not limited to such examples. It is obvious that various examples of variations or modifications can be conceived by the person skilled in the art within the scope of the claims, and it is understood that they belong to the technical scope of the invention of course. The components in the above embodiments may be arbitrarily combined without departing from the spirit of the invention.

In Embodiments 1 and 2 described above, electronic glasses 100 or overglass 400 has a pair of electronic lenses 110 or electronic lenses 450. The present invention is not limited thereto. The electronic glasses or the overglass (the first frame of the present invention) may have, for example, only one electronic lens, or may have one lens (e.g., a prescription lens) which is not an electronic lens and one electronic lens at a time. In some cases, the electronic glasses or the overglass (the first frame of the present invention) may have three or more lenses.

In Embodiment 1 described above, as illustrated in FIG. 1 and/or the like, lens holder 200 is attached to the front side of electronic glasses 100. The present invention is not limited to this, the lens holder may be attached to the rear side of the electronic glasses. In this case, since the lens holder and the lens unit held by the lens holder are positioned between the electronic glasses and the eye of the experient, it is desirable to be formed so as to reduce the thickness of the lens holder and the lens unit.

In Embodiment 2 described above, as overglass 400 as an example of the first frame, the overglass having a shape similar to that of glasses has been described. The present invention is not limited thereto. For example, the temple may be shorter than the glasses of the experiencer, or may have a configuration without the temple. In other words, the first frame of the present invention may have only the front and lenses. According to such a configuration, it is possible to prevent a situation in which it is difficult to detach the overglass from the experient's glasses because the temple of the overglass and the temple of the experient's glasses interfere with each other. In this case, the input portion for receiving the input operation for the operation or non-operation of the liquid crystal lens may be provided, for example, on the front of the first frame.

In Embodiment 2 described above, the state in which overglass 400 is attached to experient's glasses 500 is a state in which overglass 400 is attached on the frame (front) of experient's glasses 500 by attaching portion 480. The present invention is not limited thereto. As a method of attaching the first frame of the present invention to the experient's glasses, for example, the following method may be adopted. The method that, for example, the first frame of the present invention has the clip member as the first attaching portion, and the clip member attaches to the front of the glasses from above or from below by the clip member may be adopted. The method that the temple of the first frame of the present invention is placed on, caught by, or suspended from the temple member of the experient's glasses may be adopted.

In Embodiment 2 described above, as illustrated in FIG. 13A and FIG. 13B, the case has been described in which overglass 400 is attached to the objective side of lens 503 of experient's glasses 500 when viewed from the eyes of the experiencer. The present invention is not limited thereto. The electronic lens included in the overglass may be disposed between the eyes of the experiencer and the lenses of the experient's glasses. In this case, when adjusting the position of the electronic lens in the front-back direction, it is desirable to adjust the position in the direction toward the lens of the experient's glasses (away from the eyes of the experiencer), from the viewpoint of adjusting the position of the electronic lens to a more suitable distance from the eyes of the experiencer.

In Embodiment 2 described above, the example has been described in which overglass 400 has position adjuster 490 capable of performing position adjustment in the front-back direction, the left-right direction, and the up-down direction. The present invention is not limited thereto. In the present invention, the overglass may have the position adjuster capable of performing the position adjustment in either the first direction, which is the direction in which the electronic lens approaches or moves away from the lens of the experient's glasses, or the second direction orthogonal to the first direction. That is, it may be configured be able to perform the position adjustment only in either direction of the front-back direction, the left-right direction, or the up-down direction. The first direction need not strictly coincide with the front-back direction of the overglass. The second direction is not limited to the left-right direction or the up-down direction of the overglass, it may be a direction oblique relative to the left-right direction or the up-down direction.

In Embodiment 2 described above, one position adjuster 490 performs position adjustment in all directions of the front-back direction, the left-right direction, and the up-down direction. The present invention is not limited to this. In the present invention, position adjusters different for each direction may be provided. That is, the position adjuster for the front-back direction, the position adjuster for the left-right direction, and the position adjuster for the up-down direction, may be provided separately, respectively. In the case where the position adjusters for each direction are provided separately, the respective position adjusters may be provided at different positions of the eyewear of the present invention. More specifically, for example, the position adjusters for the left-right direction and the up-down direction may be provided in the vicinity of the bridge, and the position adjuster for the front-back direction may be provided at the end piece (the portion between the front and the temple).

In Embodiment 1 described above, electronic glasses 100 and lens holder 200 are formed separately. Similarly, in Embodiment 2 described above, overglass 400 and experient's glasses 500 were formed separately. The present invention is not limited thereto. For example, the eyewear system of the present invention may be an eyewear system in which two lenses of a first lens having an optical characteristic change region and a second lens having a predetermined diopter are held in a state of being overlapped each other in one frame. In this case, it is desirable that the position of the first lens is adjustably configured relative to the second lens. With such a configuration, in the eyewear system in which the second lens has a diopter suited to the visual acuity of the experiencer, the field of view through both the first lens and the second lens can be experienced by the experiencer by changing the refractive index of the optical characteristic change region of the first lens. The optical characteristic change region of the first lens can be positioned at the position suitable for the experiencer, by adjusting the position of the first lens relative to the second lens. For this reason, even in the case where there is no glasses suited to the visual acuity of the experiencer, the eyewear system of the present invention can be suitably experienced by the experiencer having weak visual acuity.

Electronic glasses 100 according to Embodiment 1 or overglass 400 according to Embodiment 2 may be provided with a notifying unit (not illustrated) that receives an operation on control unit 150 (460) and notifies the user or an operator (a person other than the user who supports trial use of the user and/or the like) of the change in the refractive index of electronic lens 110 (liquid crystal lens 450a) by an LED or sound when the refractive index of electronic lens 110 (liquid crystal lens 450a) changes. With such a configuration, the user or the operator who tries to use electronic glasses 100 according to Embodiment 1 or overglass 400 according to Embodiment 2 can recognize the change in the refractive index of electronic lens 110 (liquid crystal lens 450a) based on light or sound other than the change in the refractive index.

This application is entitled to and claims the benefit of Japanese Patent Application No. 2018-020329 filed on February 7, 2018 and Japanese Patent Application No. 2018-197410 filed on October 19, 2018, the disclosure of which including the specification, drawings and abstract is incorporated herein by reference in its entirety.

### Industrial Applicability

The present invention is suitable as an eyewear system capable of easily experiencing eyewear having a lens including a region in which optical characteristics change.

### Reference Signs List

1 Eyewear system
100 Electronic glasses
110 Electronic lens
111 First region
112 Second region
120 Frame
130 Front
131 Rim
132 Bridge
133 Nasal pad
134 End piece
140 Temple
141 Hinge
142 Ear hooking portion
143 Input portion
150 Control unit
160 Battery
200 Lens holder
210 Holder
211 Outer frame portion
2112 Arm portion
212 First supporting portion
213 Second supporting portion
220 Position adjuster
221 Arm holder
222 Beam portion
223 Gear portion
224 first movable portion
225 Second movable portion
230 Attaching portion
231 Connecting portion
232 Shaft member
233 Upward claw portion
234 Attaching beam portion
2341 Gripping portion
235 Spring
236 Downward claw portion
300 Lens unit
300 Appropriate lens unit
300 Lens unit
310 Lens
320 Frame portion
330 Knob portion
400 Overglass
410 Front
411 Rim
412 Bridge
420 Temple
430 Frame
440 Input portion
450 Electronic lens
450a Liquid Crystal Lens
460 Control unit
470 Power source
480 Attaching portion
480P portion
490 Position adjuster
500 Experient's glasses
501 Front
502 Temple
503 Lens

## Claims

1. An eyewear system, comprising:
a first lens including an optical characteristic change region where optical characteristics change;
a first frame holding the first lens; and
a second frame holding a second lens different from the first lens,
wherein the first frame has a first attaching portion for attaching the first frame to the second frame, or the second frame has a second attaching portion for attaching the second frame to the first frame, such that the first lens and the second lens face each other, and the optical characteristic change region is located within a field of view of an experiencing person through the second lens.

2. The eyewear system according to claim 1, wherein the second frame has the second attaching portion for attaching the second frame to the first frame from an objective side of the first lens, and the second frame has a second lens position adjuster for adjusting a relative position of the second lens relative to the first lens of the first frame in a state where the second frame is attached to the first frame.

3. The eyewear system according to claim 2, wherein the second lens position adjuster further comprises a second lens angle adjuster for adjusting an angle of a lens surface of the second lens such that the lens surface of the second lens faces a lens surface of the first lens.

4. The eyewear system according to claim 3, wherein the second lens angle adjuster has a first movable center along the up-down direction of the first frame, and changes an angle of the lens surface of the second lens around the first movable center.

5. The eyewear system according to claim 3 or 4, wherein the second lens angle adjuster has a second movable center along the left-right direction of the first frame, and changes an angle of the lens surface of the second lens around the second movable center.

6. The eyewear system according to any one of claims 1 to 5, wherein the second frame holds a plurality of the second lenses in an overlapped state.

7. The eyewear system according to claim 6, wherein the second frame holds a plurality of the second lenses in a state being closely attached or close to each other.

8. The eyewear system according to any one of claims 1 to 7, wherein the second lens is any one of a myopic lens, a hyperopic lens, an astigmatic lens, a color lens, a polarized lens, a light control lens, an antidazzle lens, or a specific wavelength cut lens.

9. The eyewear system according to any one of claims 1 to 8, wherein the second frame holds the second lens rotatably about a central axis of the second lens.

10. The eyewear system according to claim 1, wherein the first frame has the first attaching portion for attaching the first frame to the second frame from an objective side of the second lens, and the first frame has a first lens position adjuster for adjusting a relative position of the first lens relative to the second lens of the second frame in a state where the first frame is attached to the second frame.

11. The eyewear system according to claim 10, wherein the first lens position adjuster adjusts the relative position of the first lens toward or away from the second lens of the second frame and/or in a second direction orthogonal to the first direction.

12. The eyewear system according to claim 11, wherein the second direction is the up-down direction or the front-back direction of the second frame.

13. The eyewear system according to any one of claims 10 to 12, wherein the first lens position adjuster moves the first lens and another component of the first frame except for the first attaching portion, when adjusting the position of the first lens.

14. The eyewear system according to any one of claims 1 to 13, wherein the optical characteristic change region is an electrical element whose optical characteristics change by electrical control.

15. The eyewear system according to any one of claims 1 to 14, further comprising a control unit for controlling the electrical element, the control unit being provided in the first frame or the second frame.

16. The eyewear system according to any one of claims 1 to 15, further comprising an input portion for receiving an input operation instructing operation or non-operation of the electric element to the control unit, the input portion being provided in the first frame or the second frame.

17. The eyewear system according to any one of claims 10 to 16, wherein the second lens has a predetermined diopter.

18. The eyewear system according to any one of claims 1 to 17, wherein the first attaching portion or the second attaching portion is a clip-shaped member biased in a closing direction.
